# FASCICULE DE BREVET EUROPEEN

(11) **EP 0 538 152 B1**
(45) Date de publication et mention de la délivrance du brevet: **02.08.1995**
(21) Numéro de dépôt: 92420337.5
(22) Date de dépôt: 29.09.1992
(51) Int. Cl.: A61B 17/14

(54) **Ancillaire pour la mise en place d'une prothèse de plateau tibial**
Hilfsinstrument zum Einsetzen einer Tibiaplateau-Prothese
Ancillary equipment for implanting a prosthetic tibial plateau

(30) Priorité: 01.10.1991 FR 9112284
(43) Date de publication de la demande: 21.04.1993
(73) Titulaire: IMPACT, F-01800 Charnoz (FR); Collomb, Jean, F-26800 Portes Les Valence (FR); Majou, Claude, F-01800 Meximieux (FR)
(72) Inventeur: Collomb, Jean, F-26800 Porte Les Valence (FR); Majou, Claude, F-01800 Meximeiux (FR)
(74) Mandataire: Laurent, Michel

(56) Documents cités:
- EP-A- 0 327 249
- EP-A- 0 337 901
- US-A- 4 952 213

## Description

L'invention concerne un ancillaire, permettant la mise en place d'une prothèse pour plateau tibial.

Le plateau tibial constitue l'extrémité supérieure du tibia, sur lequel viennent s'articuler et prendre appui les condyles du fémur ; l'ensemble, avec la rotule, constitue l'articulation du genou.

Lorsque l'on procède à la mise en place d'une prothèse tricompartimentale de genou, il s'avère nécessaire de procéder également au remplacement du plateau tibial, compte tenu de l'état pathologique du genou du patient à opérer.

La mise en place d'une prothèse pour plateau tibial suppose préalablement un travail préparatoire effectué par le praticien, afin que cette prothèse soit adaptée correctement au tibia considéré.

A ce jour, différents appareillages ont été proposés pour effectuer ce travail préparatoire. Ils sont souvent constitués d'une tige intra-médullaire, destinée à être insérée dans le canal médullaire du tibia, ou le plus généralement d'une tige de visée externe, destinée à favoriser l'orientation des découpes à effectuer au niveau du tibia. Néanmoins, aucun des appareillages connus à ce jour ne donne satisfaction quant à la précision des orientations obtenues et, par voie de conséquence, quant aux découpes effectuées préalablement à la mise en place de la prothèse de plateau tibial.

Le préambule de la revendication indépendante mentionne les caractéristiques connues du document US-A-4952213 qui décrit une ancillaire pour la mise en place d'une prothèse du plateau tibial comprenant une tige intra-médullaire et d'autres tiges ajustables en longueur et en angle de rotation.

L'invention vise à pallier les inconvénients mentionnés ci-dessus. Elle propose un ancillaire pour la mise en place d'une prothèse de plateau tibial, qui soit à la fois simple à mettre en oeuvre, permette d'aboutir à des découpes précises du tibia, sans pour autant nécessiter un appareillage lourd d'utilisation.

Cet ancillaire pour la mise en place d'une prothèse du plateau tibial, comprend :
- une tige intra-médullaire insérée dans le canal médullaire du tibia ;
- une tige de visée externe, parallèle à la tige intra-médullaire, maintenue à ses deux extrémités au niveau du tibia.

Cet agensement permet au praticien de réaliser une visée intramédullaire seule, une visée extramédullaire seule, ou bien une visée combinant les deux.

Cet ancillaire comprend en outre :
- un guide de coupe tibiale, de forme sensiblement arquée et donc anatomique, positionné au niveau de la partie supérieure du tibia, percé sur ses deux faces planes principales d'au moins trois orifices traversants, dont l'un est destiné à recevoir la tige de visée externe, et comportant sur ses deux faces incurvées :
   . une pluralité d'orifices traversants, destinés à permettre la fixation du guide de coupe tibiale au niveau du tibia ;
   . une fente traversante, s'étendant parallèlement aux faces planes principales, et destinée à guider une scie lors de la découpe du plateau tibial à remplacer ;
- un palpeur, constitué par une tige linéaire recourbée à ses deux extrémités, et munie d'une vis destinée à venir se positionner dans l'un des orifices du guide de coupe tibiale adjacents à celui recevant la tige de visée externe.

En d'autres termes, l'invention consiste à prévoir un guide de coupe tibiale, fixé au niveau de la tige de visée externe, et réalisé de telle sorte qu'il vienne prendre appui contre le tibia dont on désire remplacer la surface articulaire généralement dénommée plateau tibial, et qui est muni d'un palpeur afin de permettre le positionnement précis dudit guide en fonction de l'usure détectée dudit plateau tibial, et ceci tout en cherchant à effectuer la coupe minimum, de sorte à utiliser une pièce prothétique la moins épaisse possible.

Selon l'invention, le guide de coupe se prolonge davantage d'un coté que de l'autre, afin d'épouser au mieux le côté interne sans entrer en conflit avec le paquet rotulien externe.

Selon une forme particulièrement avantageuse de l'invention, le guide de coupe est symétrique par rapport au plan médian horizontal, de telle sorte qu'il est réversible et peut être utilisé pour le côté droit comme pour le côté gauche.

Avantageusement, le palpeur comporte une lumière ménagée dans la tige linéaire et alignée avec celle-ci, destinée à coulisser avec un ajustement dit "gras" ; les deux extrémités peuvent être utilisées alternativement :
- l'une indiquant le côté pathologique ;
- l'autre indiquant le côté sain.

La manière dont l'invention peut être réalisée et les avantages qui en découlent, ressortiront mieux de l'exemple de réalisation qui suit, donné à titre indicatif et non limitatif, à l'appui des figures annexées.

La figure 1 est une représentation schématique de l'ancillaire conforme à l'invention en place au niveau d'un tibia.

La figure 2 est une représentation schématique de la face supérieure ou inférieure plane du guide de coupe tibiale conforme à l'invention, dont la figure 3 est une coupe transversale.

La figure 4 est une représentation schématique du palpeur de l'ancillaire conforme à l'invention, dont la figure 5 est une vue de dessus.

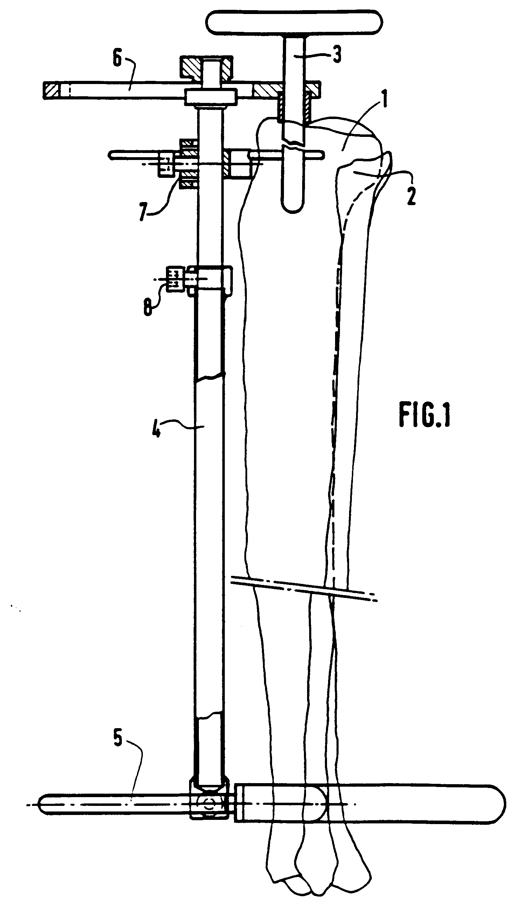

On a représenté sur la figure 1 l'ancillaire conforme à l'invention en place sur un tibia (1). On a également représenté le péroné (2). Cet ancillaire comprend fondamentalement une tige intra-médullaire (3), insérée dans le canal médullaire du tibia. Il comprend également une tige de visée externe (4), parallèle à la tige intra-médullaire (3), et maintenue à l'extérieur du tibia au moyen de deux supports, respectivement (5,6). Le support (6) est percé d'une lumière, permettant un réglage précis de la tige de visée externe par rapport au tibia. En outre, la longueur de cette tige (4) peut être réglée au moyen d'une molette portant la référence (8).

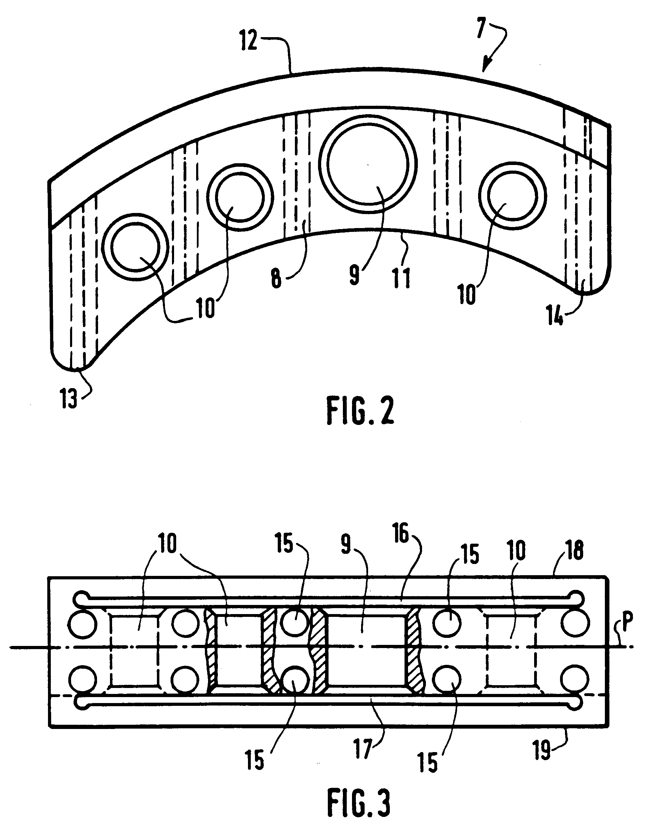

Cette tige de visée externe est destinée à recevoir un guide de coupe tibiale (7), plus particulièrement représenté dans les figures 2 et 3. Ce guide de coupe tibiale (7) est un élément métallique de forme arquée, comportant deux faces principales (18,19) planes et deux faces incurvées respectivement (11) et (12). Les faces planes (18) et (19) sont percées d'orifices traversants (9,10) de diamètres différents. L'orifice (9) est destiné à recevoir la tige de visée externe (4). En revanche, les orifices (10) situés de part et d'autre de cet orifice (9), sont destinés à recevoir le palpeur (20) du guide de coupe tibiale, décrit plus en détail ultérieurement.

Comme on peut le voir sur la figure 2, le guide de coupe est disymétrique, l'un des côtés se prolongeant davantage que l'autre. Le côté le plus allongé (13) correspond au côté interne, c'est-à-dire qui vient se placer vers l'intérieur de l'articulation lorsqu'on lui fait face. L'autre côté constitue le côté externe (14) de l'articulation.

Selon une autre caractéristique de l'invention, les faces incurvées (11) et (12) sont également percées d'orifices traversants portant la référence (15), rassemblés en cinq jeux de deux orifices, chacun des jeux étant orienté rectilignement et parallèlement aux faces planes (18,19). En outre, lesdites faces latérales incurvées (11) et (12) comportent deux fentes également traversantes (16) et (17), parallèles aux faces planes (18,19), destinées, comme il sera décrit plus en détail ultérieurement, à permettre le passage et le guidage d'une scie pour procéder aux coupes du tibia.

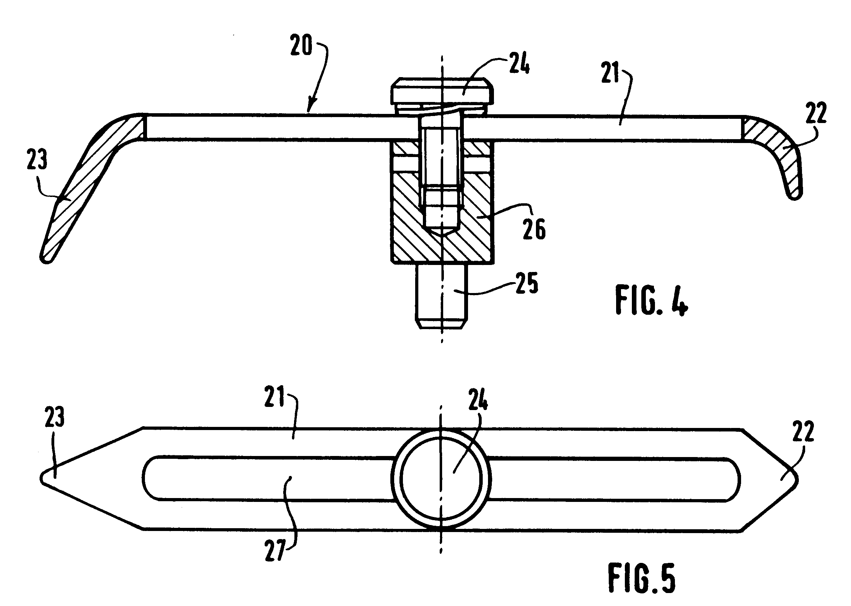

On a représenté sur les figures 4 et 5 une représentation du palpeur (20) du guide de coupe tibiale. Ce palpeur est essentiellement constitué d'une lame métallique rectiligne (21), percée d'une lumière (27) alignée avec la dimension principale dudit palpeur,dans laquelle peut se mouvoir un support (26) associé à une vis (24), une goupille (25) étant associée audit support (26). La goupille (25) est destinée à venir s'insérer dans les orifices (10) du guide de coupe tibiale décrit précédemment. Le support (26) associé à la vis (24) est destiné à déplacer la goupille (25) par rapport au palpeur (20).

Corrélativement, les deux extrémités de la lame métallique (21) sont recourbées, respectivement (22) et (23), l'extrémité (23) étant de dimensions plus importantes que l'extrémité (22). Ces extrémités (22) et (23) constituent les points de palpage proprement dits, l'extrémité (22) correspondant au côté sain du plateau tibial, et l'extrémité (23) correspondant au côté effondré également dénommé côté pathologique du plateau tibial.

Il va être décrit plus en détail le mode de fonctionnement de l'ancillaire conforme à l'invention. Une fois la tige médullaire (3) insérée dans le canal médullaire du tibia (1), on met en place les supports (5) et (6) de la tige de visée externe (4), puis on procède à la mise en place de ladite tige (4) après avoir inséré le guide de coupe tibiale (7) par l'intermédiaire de l'orifice (9) au niveau de la tige. On règle alors le positionnement de la tige de visée externe (4), de telle sorte que la face concave latérale (11) du guide de coupe (7) vienne en contact avec le tibia. Puis, on règle la hauteur du guide de coupe tibiale (7) en positionnant le palpeur (20) dans les différents orifices (10) selon le côté sain, puis pathologique du plateau tibial, la position relative des points de palpage proprement dits (22,23) pouvant être adapté en déplaçant le support (26) de la goupille (25) dans la lumière (27), afin de pouvoir procéder au calage du guide de coupe (7).

Le palpeur (20) est utilisé successivement en partie externe et en partie interne, où l'on choisit l'un des deux orifices (10) en fonction de la taille du genou du patient. Ce double palpage permet de définir la quantité d'os à couper en épaisseur.

Une fois le réglage en hauteur du guide de coupe tibiale (7) réalisé, on fixe ce dernier dans le tibia en insérant des broches dans les orifices (15) situés sur les parois latérales (11) et (12) dudit guide de coupe. On ôte alors la tige de visée externe en la faisant coulisser au travers de l'orifice (9), ainsi que la tige intramédullaire (3), et l'on procède à la découpe du plateau tibial dans la fente supérieure (16) dudit guide de coupe dans le tibia, les dites broches jouant outre le rôle de fixation du guide de coupe tibiale (7) dans le tibia (1), le rôle de sécurité, guidant la lame de scie horizontalement. La prothèse de plateau tibial peut alors être mise en place, après que l'on ait formé son emplacement au moyen de quilles de formage, et jugé de sa taille grâce à des plateaux d'essai.

Comme on peut le voir sur la figure 3, le guide de coupe est symétrique par rapport au plan médian. De la sorte, il est réversible et peut être avantageusement utilisé pour l'articulation droite comme pour l'articulation gauche du genou, il suffit pour ce faire, de le retourner.

Du fait de sa grande simplicité de réalisation, et de sa grande simplicité de mise en oeuvre, cet ancillaire s'avère particulièrement adapté pour la mise en place de prothèse pour plateau tibial, ce que les ancillaires connus à ce jour ne permettaient pas d'obtenir.

## Revendications

1. Ancillaire pour la mise en place d'une prothèse du plateau tibial, comprenant :
- une tige intra-médullaire (3) insérée dans le canal médullaire du tibia (1) à opérer ;
- une tige de visée externe (4), parallèle à la tige intra-médullaire (3), maintenue à ses deux extrémités (5,6) au niveau du tibia (1) :
- un guide de coupe tibiale (7), de forme sensiblement arquée, définissant deux faces latérales incurvées (11,12), limitées par deux faces planes principales (18, 19), et comportant sur ses deux faces incurvées (11,12) :
. une pluralité d'orifices traversants (15), destinés à permettre la fixation du guide de coupe tibiale (7) au niveau du tibia (1) ;
. une fente traversante (16,17), s'étendant parallèlement aux faces planes principales (18,19), et destinée à guider une scie lors de la découpe du plateau tibial à remplacer ;
- un palpeur (20), constitué par une tige linéaire (21) recourbée à ses deux extrémités (22,23), et munie d'une goupille (25), caractérisé en ce que lesdites faces planes principales (18,19) sont percées d'au moins trois orifices traversants (9,10), dont l'un (9) est destiné à recevoir la tige de visée externe (4), et un ce que ladite goupille (25) est destinée à venir se positionner dans l'un des orifices (10) du guide de coupe tibiale (7) adjacents à celui (9) recevant la tige de visée externe (4).

2. Ancillaire selon la revendication 1, caractérisé en ce que le guide de coupe tibiale (7) se prolonge davantage d'un coté que de l'autre, afin de former côté interne (13), venant se placer au contact du tibia.

3. Ancillaire selon l'une des revendications 1 et 2, caractérisé en ce que le guide de coupe tibiale (7) est symétrique par rapport à un plan médian horizontal P, de telle sorte qu'il est réversible et peut être utilisé pour le côté droit comme pour le côté gauche.

4. Ancillaire selon la revendication 3, caractérisé en ce que les faces latérales incurvées (11,12) comportent une pluralité de deux jeux d'orifices traversants (15) pour la fixation du guide de coupe tibiale sur le tibia, et comporte deux fentes (16,17), parallèles aux faces planes principales (18,19), pour le guidage d'une scie de coupe du plateau tibial à remplacer.

5. Ancillaire selon l'une des revendications 1 à 4, caractérisé en ce que le palpeur (20) comporte une lumière (27) ménagée dans la tige linéaire (21) et alignée avec celle-ci, destinée à recevoir une vis (24) associé au support (26) de la goupille (25), le dit support (26) étant susceptible de se déplacer dans la dite lumière (27), et d'y être fixé réversiblement au moyen de la vis (24).

## Claims

1. Ancillary equipment for implantation of a prosthetic tibial plateau, comprising :
- an intramedullary stem (3) inserted into the medullary channel of the tibia (1) to be operated on ;
- an external sighting stem (4), parallel to the intramedullary stem (3), held at both its ends (5, 6) level with the tibia (1) ;
- a tibial cutting guide (7), substantially arcuate in shape, defining two lateral inwardly-curved faces (11, 12), limited by two main plane faces (18, 19), and comprising on its two inwardly-curved faces (11, 12) :
. a plurality of transverse apertures (15) intended to permit the tibial cutting guide to be secured at the level of the tibia (1) ;
. a transverse slot (16, 17) extending parallel to the main plane faces, and intended to guide a saw during incision of the tibial plateau to be replaced ;
- a tracer (20), constituted by a linear stem (21) curved at both its ends and fitted with a pin (25),
characterised in that the said main plane faces (18, 19) are transversed by at least three transverse apertures (9, 10), of which one (9) is intended to receive the external sighting stem (4), and in that the said pin (25) is intended to be positioned in one of the apertures (10) of the tibial cutting guide (7) adjacent to that (9) which receives the external sighting stem (4).

2. Ancillary equipment according to claim 1, characterised in that the tibial cutting guide (7) is longer on one side than on the other, in order to form an internal side (13) which is placed in contact wih the tibia.

3. Ancillary equipment according to one of claims 1 and 2, characterised in that the tibia] cutting guide (7) is symmetrical with respect to a horizontal medial plane P, so that it is reversible and may be used for the right side as well as for the left side.

4. Ancillary equipment according to claim 3, characterised in that the inwardly-curved lateral faces (11, 12) comprise a plurality of two sets of transverse apertures (15) for fixing the tibia] cutting guide on the tibia, and comprise two slots (16, 17) parallel to the main plane faces (18, 19) in order to guide a saw cutting the tibial plateau to be replaced.

5. Ancillary equipment according to one of claims 1 to 4, characterised in that the tracer (20) comprises an aperture (27) provided in the linear stem (21) and aligned with the latter, intended to receive a screw (24) associated with the support (26) of the pin (25), said support (26) being capable of movement in the said aperture (27) and of being reversibly secured there by means of the screw (24).

## Patentansprüche

1. Hilfsinstrument zum Einsetzen einer Prothese für die Kniegelenkfläche des Schienbeins,
- mit einem intramedullären Stab (3), der in den Knochenmarkkanal des zu behandelnden Schienbeins (1) eingesetzt wird;
- mit einem äußeren, zu dem intramedullären Stab (3) parallel verlaufenden Stellstab (4), der an seinen beiden Enden (5, 6) im Bereich des Schienbeins (1) gehalten wird;
- mit einer Schienbeinschneideführung (7) von leicht gekrümmter Form, die zwei gebogene Seitenflächen (11, 12) definiert, die wiederum durch zwei ebene Hauptflächen (18, 19) begrenzt werden, und wobei die beiden gebogenen Flächen (11, 12) folgendes aufweisen:
. eine Vielzahl von durchgehenden Öffnungen (15), die für die Befestigung der Schienbeinschneideführung (7) im Bereich des Schienbeins (1) vorgesehen sind;
. einen durchgehenden Schlitz (16, 17), der sich parallel zu den ebenen Hauptflächen (18, 19) erstreckt und der dazu vorgesehen ist, eine Säge während des Abschneidens der zu ersetzenden Kniegelenkfläche des Schienbeins zu führen;
- sowie mit einem Taster (20), der aus einem geradlinigen Stab (21) besteht, der an seinen beiden Enden (22, 23) abgebogen ist, der mit einem Stift (25) versehen ist, dadurch gekennzeichnet, daß die ebenen Hauptflächen (18, 19) von mindestens drei durchgehenden Öffnungen (9, 10) durchdrungen sind, von denen eine (9) dazu vorgesehen ist, den äußeren Stellstab (4) aufzunehmen, und daß der Stift (25) dazu vorgesehen ist, in eine der Öffnungen (10) der Schienbeinschneideführung (7) eingesetzt zu werden, die benachbart derjenigen (9) sind, die den äußeren Stellstab (4) aufnimmt.

2. Hilfsinstrument nach Anspruch 1, dadurch gekennzeichnet, daß eine Seite der Schienbeinschneideführung (7) länger als die andere ausgebildet ist, wodurch eine Innenseite (13) gebildet wird, die zur Anlage mit dem Schienbein kommt.

3. Hilfsinstrument nach einem der Ansprüche 1 oder 2, dadurch gekennzeichnet, daß die Schienbeinschneideführung (7) bezüglich einer horizontalen Mittelebene P symmetrisch ist, so daß diese umkehrbar ist und sowohl für die rechte als auch für die linke Seite verwendet werden kann.

4. Hilfsinstrument nach Anspruch 3, dadurch gekennzeichnet, daß die gebogenen Seitenflächen (11, 12) eine Vielzahl von Zweiersätzen an durchgehenden Öffnungen (15) für die Befestigung der Schienbeinschneideführung am Schienbein aufweisen, und ferner zu den ebenen Hauptflächen (18, 19) parallel verlaufende Schlitze (16, 17) für die Führung einer Säge zum Abschneiden der zu ersetzenden Kniegelenkfläche des Schienbeins aufweisen.

5. Hilfsinstrument nach einem der Ansprüche 1 - 4, dadurch gekennzeichnet, daß der Taster (20) ein in dem geradlinigen Stab (21) ausgespartes und mit diesem ausgerichtetes Schlitzloch (27) aufweist, das dazu vorgesehen ist, eine Schraube (24) aufzunehmen, die einem Träger (26) des Stifts (25) zugeordnet ist, wobei der Träger (26) im Schlitzloch (27) verschiebbar ist und dort lösbar mittels der Schraube (24) festgestellt werden kann.
